# EUROPEAN PATENT APPLICATION

(11) **EP 3 489 660 A1**
(43) Date of publication of application: **29.05.2019**
(21) Application number: 17204066.9
(22) Date of filing: 28.11.2017
(51) Int. Cl.: G01N 21/51, G01N 21/59, F25C 1/00, F25D 29/00

(54) **SYSTEM FOR DETECTING AND MANAGING A CHANGE OF STATE**

(71) Applicant: Vestel Elektronik Sanayi ve Ticaret A.S., 45030 Manisa (TR)
(72) Inventor: SARIARSLAN, Alper, 45030 Manisa (TR)
(74) Representative: Ascherl, Andreas

(57) **Abstract**

A system (1) for detecting a change of a state (2) of a matter contained in a container (3). The system comprising a holder (4) for holding the container (3), a light emitting source (5) placed on or in proximity of a first wall (6) of the holder (4), the light emitting source (5) is adapted to generate a light (7),an optical sensor (8) placed on or in proximity of a second wall (9) of the holder (4), different from the first wall (6), such that the light emitting source (5) and the optical sensor (8) faces each other while in action, and adapted to receive the light (7) from the light emitting source (5) after passing through the container (3) and to generate a lighting data (10) based on the light (7) received, wherein the container (3) is adapted to be placed in the holder (4), such that transparent parts of the container (3) faces the light emitting source (5) and the sensor (8), and a processor (11) adapted to receive the lighting data (10) and adapted to determine, if the state of the matter has changed. The system may be used in a cooling device (17), such as a refrigerator, for regulating the cooling mechanism such that in a specific compartment water is cooled, but not changed to ice, and in another specific compartment water is kept to change its state to form ice.

## Description

The present invention relates to a system for detecting a change of a state of a matter contained in a container according to claim 1 of the present disclosure, and to a cooling device adapted to cool a matter according to claim 6 of the present disclosure.

### Background of the Invention

A change of a state of matter and to retain a state of matter without change is required for various human needs, or any other industrial purpose. However, if such a state of a matter is not retained when desired, or if a change of a matter does not occur as desired, this may lead to an inconvenience and sometimes even to losses.

One specific problem refers to a refrigerator, where specific compartments are provided for cooling water, while not changing it into ice, and another specific compartment is provided where the water is kept to change its state to form ice. In case that either of these functionalities are not achieved it becomes inconvenient to the user, as a specific performance by the refrigerator is not achieved. When, in the compartment for just cooling the water, it is cooled to a level which results in a change of its state to ice, it results in breaking the container containing the water due to the conversion from water to ice which has a higher density. On the other hand, in the compartment which is intended for converting the water into the ice, and if the conversion does not occur, it becomes inconvenient to the user, as he shall not be having the ice when required.

Hence, there exists a need for determining a change of a matter, when it is kept in a container to either change the state or retain a current state.

### Object of the Invention

It is therefore an object of the present invention to provide a system for determining a change in state of a matter kept in a container.

### Description of the Invention

The aforementioned object is achieved by a system for detecting a change of a state of a matter contained in a container according to claim 1 of the present invention.
The system for detecting a change of a state of a matter contained in a container comprises a holder for holding the container, a light emitting source placed on or in proximity of a first wall of the holder, wherein the light emitting source is adapted to generate a light, an optical sensor placed on or in proximity of a second wall of the holder different from the first wall such that the light emitting source and the sensor faces each other while in action, wherein the sensor is adapted to receive the light from the light emitting source after passing through the container and to generate a lighting data based on the received light, wherein the container is adapted to be placed in the holder such that transparent parts of the container face the light emitting source and the sensor, and a processor adapted to receive the lighting data and adapted to determine, if the state of the matter has changed.
This embodiment is beneficial, because it provides an efficient technique for determining a change in the state of the matter by using optical effects, since in particular ice and water have a different transparency.

Further embodiments of the present invention are subject of the further subclaims and of the following description, referring to the drawings.

According to a preferred embodiment of the system, the processor is adapted to generate an alarm trigger based on the determination of the state of the matter, the system comprising an alarm adapted to receive the alarm trigger from the processor, and adapted to generate an indication indicating the change of the state of the matter.

This embodiment is beneficial, as it provides a mechanism to alarm the user of the system, if the change of the state of the matter is not as desired. This helps the user to further take appropriate steps to deal with such scenario.

According to a further preferred embodiment of the system, the processor is adapted to generate a control trigger based on the determination of the state of the matter, the system comprising a controller adapted to receive the control trigger and adapted to control the change of the state of the matter.

This embodiment is beneficial, as it enables the system to take a corrective step automatically, to correct the situation by controlling the change of the state of the matter as desired.

According to another embodiment of the system, the matter is adapted to be transparent in a first state and to be translucent or opaque in a second state.

This embodiment is beneficial, as it creates a clear and optical differentiation between two states of the matter. This leads to reduction in false positives.

According to a further embodiment of the system, the first state is a liquid state, and the second state is the solid state.

This embodiment is beneficial, as it provides another efficient implementation for the matter like water, which is transparent in liquid state and translucent or almost opaque in solid state.

The before mentioned object is also solved by a cooling device adapted to cool a matter according to claim 6.

According to an embodiment of the invention, the cooling device further comprises a compartment having the holder for keeping the container which comprises the matter and a cooling mechanism to cool the matter contained in the container, wherein the matter is defined to have a physical property to change the state when cooled beyond a particular temperature.

This embodiment is beneficial, as it provides an implementation of the system in cooling devices, so that the state of the matter can be determined while carrying out cooling function onto the matter.

According to a further preferred embodiment of the invention, the cooling device comprises at least two compartments, namely a cooling compartment and a state change compartment, wherein the cooling compartment is adapted to be cooled in a way by the cooling mechanism so that the state of the matter does not change, and the state change compartment is adapted to be cooled in a fashion so that the change of the state of the matter occurs within a predefined time interval.

This embodiment is beneficial, as it provides implementation of the system in cooling devices like refrigerators, which has two compartments, one to cool the matter up to a level to retain current state of matter, and another compartment to cool the matter to change the state in a predefined time interval, in particular from water into ice. Hence, the technique for determining the change of the state of the matter can be implementable in both the compartments or either of them.

According to another embodiment of the invention, the processor is adapted to generate a first alarm trigger, if the state of the matter has changed in the cooling compartment, and the alarm is adapted to receive the first alarm trigger from the processor and adapted to generate an indication indicating the change of the state of the matter.

This embodiment is beneficial, as it facilitates implementation of an alarm in the cooling compartment, in which the state of the matter should not change. Hence, helping a user to take necessary steps, if the state of the matter changes.

According to a further embodiment of the cooling device, the processor is adapted to generate a second trigger alarm, if the state of the matter inside the state change compartment does not change in the predefined time interval, and the alarm is adapted to receive the second alarm trigger from the processor, and adapted to generate an indication indicating no change in the matter in the predefined interval.

This embodiment is beneficial, as it facilitates implementation of an alarm in the state change compartment, in which the state of the matter should change in a predefined time interval. Hence, this helps a user to take necessary steps, if the state of the matter does not change in the predefined time interval.

According to a further preferred embodiment of the cooling device, the processor is adapted to generate a first control trigger, if the state of the matter has changed in the cooling compartment, and the controller is adapted to receive the first control trigger and adapted to regulate the cooling mechanism to reverse the change of the state of the matter.

This embodiment is beneficial, as it facilitates controlling of the cooling mechanism in the cooling compartment, if the state of matter is changes, where such change of the matter is not required, and can be rather problematic. The controller may trigger for even reverse change of the state of the matter to the initial state.

According to another embodiment of the cooling device, the processor is adapted to generate a second control trigger, if the state of the matter inside the state change compartment does not change in the predefined time interval, and the controller is adapted to receive the second control trigger, and adapted to regulate the cooling mechanism to enhance cooling inside the state change compartment to fasten the change of state of the matter.

This embodiment is beneficial, as it facilitates controlling the cooling mechanism in the state change compartment, if the state of the matter does not change in the predefined time interval. This is carried out automatically, without human intervention, and mostly by enhancing the cooling effect from the cooling mechanism. However, any other means can be used by the cooling device to facilitate the change of the state of the matter in the state change compartment.

Further benefits, goals and features of the present invention will be described by the following specification of the attached figures, in which components of the invention are exemplarily illustrated. Components of the system and the method according to the invention, which match at least essentially with respect to their functions, can be marked with the same reference sign, wherein such components do not have to be marked or described in all figures.

The invention is just exemplarily described with respect to the attached figures in the following.

### Brief Description of the Drawings

Fig. 1 illustrates a schematic diagram of a system for detecting and controlling change of a state of a matter contained in a container, according to an embodiment of the invention.
Fig. 2a illustrates a holder of the Fig. 1 while the system being in function, and the matter is in first state.
Fig. 2b illustrates the holder of the Fig. 1 while the system being in function, and the matter is in second state.
Fig. 3 illustrates a schematic diagram of a cooling device for detecting and controlling change of a state of a matter contained in containers of different compartments of the cooling device, according to an embodiment of the invention.

### Detailed Description of the Drawings

The present invention focuses on detecting a change of state of a matter while being in a container which is kept in a holder. Further on detection of such change of matter, control mechanism can also be applied for reversing such change of the state. In certain environment due to natural environment or due to induced environment within the holder, state of a matter changes, and such change of the state of matter is not desired, hence detection and control of such change of matter is desired, and carried out by this invention.

While discussing the figures, references are made to the elements of other figures too.

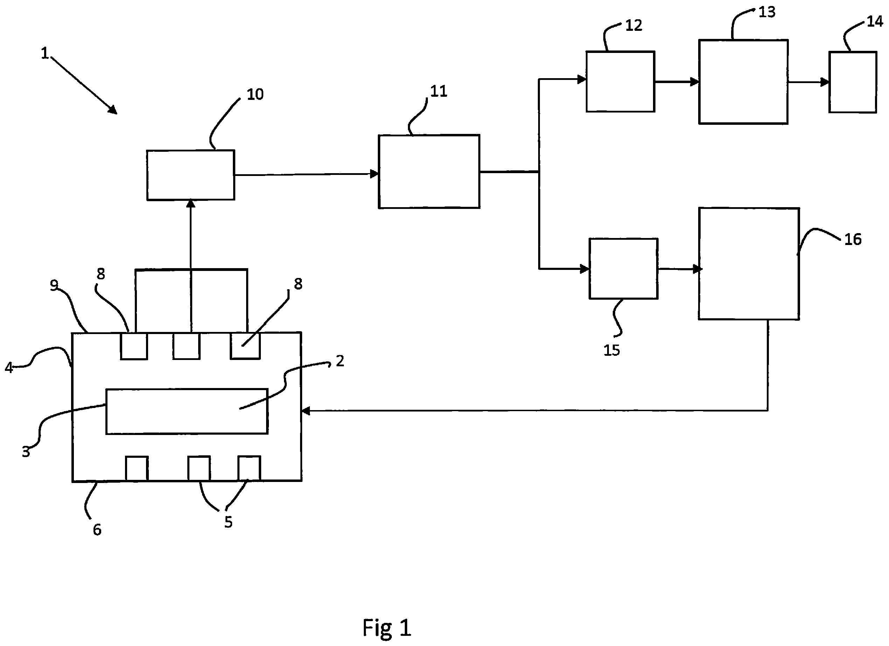

Fig. 1 illustrates a schematic diagram of a system 1 for detecting and controlling change of a state 2 of a matter, for example water, contained in a container 3. The system includes a holder 4 which holds the container 3, and a light emitting source 5 generating a light 7 and placed on a first wall 6 of the container 3. The system 1 also includes an optical sensor 8 which is placed on a second wall 9 of the holder. The first wall 6 and the second wall 9 are parallel to each other and facing each other. In an alternate embodiment, the walls 6, 9 need not be exactly parallel to each other, rather they can be at some angle to each other, whether connected to each other or not, however the placement of the light emitting source 5 and the optical sensor 8 on the walls 6, 9 shall be such that they should be facing each other, so that the optical sensor 8 can receive the light 7 emitted from the light emitting source 5, if it remains uninterrupted. The container 3 is placed within the holder 4 such that transparent parts of the container 3 faces the light emitting source 5 and the optical sensor 8. When the light emitting source 5 receives the light 7, it generates a lighting data 10.

The system 1 also includes a processor 11 which receives the lighting data 10 and determines, if the state of matter is not changed.

It is to be noted that for this system 1 to work, at least one state 2 of the matter should be transparent or translucent for allowing the light to pass through. However, the light 7 gets diffused and scattered while passing through a translucent medium such as ice.

When the state 2 of the matter changes, the level of transparency of the medium also changes, which further results in change in scattering or diffusion level of the light 7. Such change effects the lighting data 10 which varies for different level of diffusion or scattering. When such change in the lighting data 7 is identified by the processor, it determines the change of the state 2 if the matter.

The processor 11 also generates an alarm trigger 12 when it determines change in the state 2 of the matter. The system 1 also includes an alarm 13 which receives the alarm trigger 12 from the processor 11, and generates an indication 14 indicating change in the state 2 of the matter. This indication 14 can be sound, light, or any other indication mode, which can attract attention of a user. In an alternate embodiment, the alarm 13 is not required, rather system 1 just detects the change in state 2 of the matter, and may directly move on to control the change in the state 2 of the matter. In case, when the indication 14 is raised, the user can himself take any action to control the change in the state 2 of the matter.

The processor 11 also generates a control trigger 15 based on determination of the change of the state of the matter. The system 1 also includes a controller 16 which receives the control trigger 15 from the processor 11, and further uses a mechanism for controlling the change if the state 2 of the matter. Such mechanism can be to induce some change in environmental parameters, like temperature, humidity, etc., in an environment of the holder 4 in which the container 3 is placed. In one embodiment, the controller 16 is not required, rather the system just raises the indication 14 using the alarm 13, and the user can take steps to control the change of the state 2 of the matter by himself.

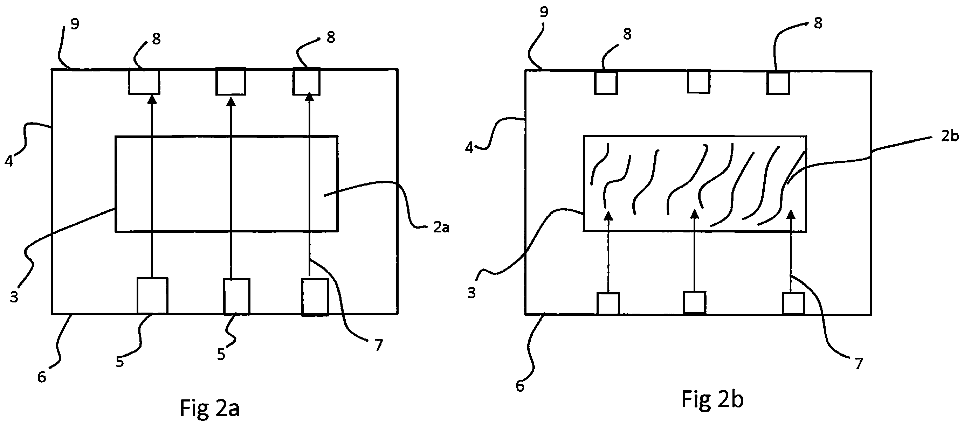

Fig. 2a illustrates the holder 4 of the system 1of Fig. 1 while the system being in function, and the matter is in first state 2a, which is transparent and allows passing of the light through the matter completely. As shown in the Fig. 2b, the light 7 is able to pass through the matter. In this case, the optical sensor 8 is able to detect the light 7 and is able to generate the lighting data 10, and the processor 11 is able to receive the lighting data 10 regularly, and determines no state change, as the lighting data received is same again and again, and no change is identified by the processor 11.

Fig. 2b illustrates the holder 4 of the system 1 of the Fig. 1 while the system being in function, and the matter changes to a second state 2b, which is opaque in nature and do not allow passing of the light 7. As shown in the Fig. 2b, the light 7 is not able to pass through the matter. In this case, the optical sensor 8 is not able to detect the light 7 and is not able to generate the lighting data 10, and the processor 11 do not receive the lighting data 10, since the matter inside the container 3 changes from the first state 2a to the second state 2b. Once, the processor stops receiving the lighting data 10 or receives the lighting data 10 of substantially scattered or diffused light 7, the processor 11 determines state change.

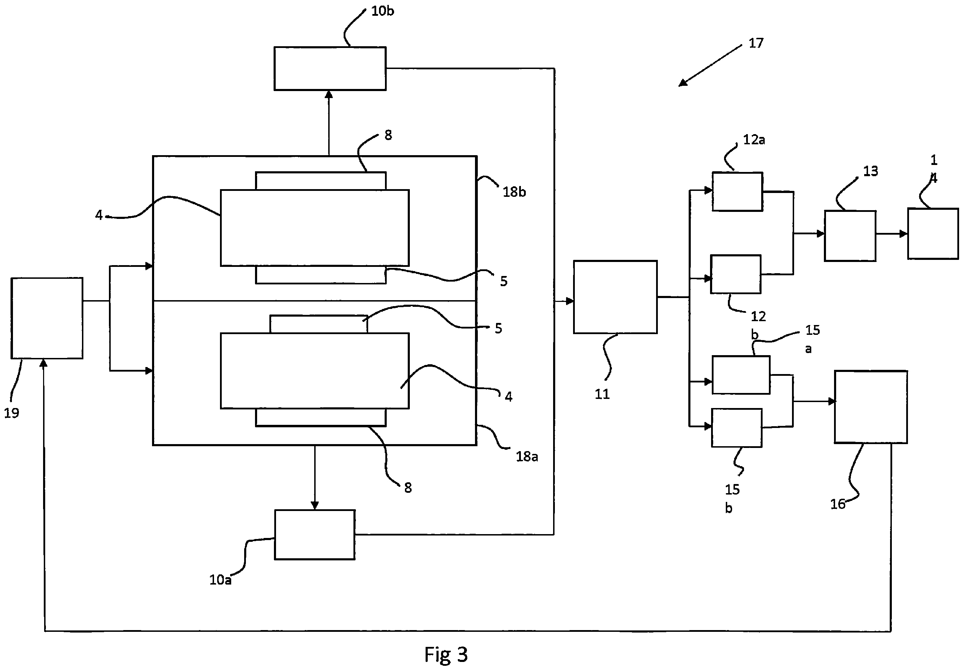

Fig. 3 illustrates a schematic diagram of a cooling device which uses the system of the Fig 1 for detecting and controlling change of the state of the matter contained in containers, which are kept in holders 4 of different compartments 18a, 18b of the cooling device17. The cooling device 17 also includes a cooling mechanism 19 which cools the matter contained in the container. The matter kept in the containers, which are kept in compartments 18a, 18b have a physical property to change the state when cooled beyond a particular temperature. One of such matter can be water.

The cooling device 17, for example a refrigerator, includes two compartments 18a, 18b. One of them is a cooling compartment 18a is cooled in such a way by the cooling mechanism 19, so that the state of the matter does not change and remains in the first state while normal functionality of the cooling device 17. Other one is a state change compartment 18b which is cooled by the cooling mechanism 19 in such a way, so that to enable change of the state of the matter from the first state to the second state.

Both the compartments 18a, 18b holders 4 have the light emitting source 5 and the optical sensor 8 placed on the walls of the holders 4. The sensors 8 are further connected to the processor 11, so that the lighting data 10a, 10b generated by each of the optical sensor 8 is sent to the processor 11 for further processing.

To function normally, the matter in the cooling compartment 18a should ensure that the matter should remain in the first state, the matter in the state change compartment 18b should change from the first state to the second state, and should remain in the second state. The processor 11 processes the lighting data 10a, 10b received from each of the compartments 18a, 18b accordingly, so that it can either trigger the alarm 13 or trigger the controller 16, if the matter in any of the compartments 18a, 18b are not in the states as desired.

In case, the matter in the first compartment 18a changes state from the first state to the second state, the processor generates a first alarm trigger 12a, which is further received by the alarm 13 to generate the indication 14 for change of state of the matter.

Similarly, when the matter in the first compartment 18a changes state from the first state to the second state, the processor generates a first control trigger 15a, which is further received by the controller 16 which further regulates the cooling mechanism to reverse the change of state of the matter.

In case, the matter in the state change compartment 18b does not change in a predefined interval, the processor generates a second alarm trigger 12b, which is further received by the alarm 13 to generate the indication for no change of the matter.

Similarly, when the matter in the state change compartment 18b does not change in a predefined interval, the processor generates a second control trigger 15b, which is further received by the controller 16 to regulate the cooling mechanism 19 for enhancing cooling inside the state change compartment 18b for fastening the change of state of the matter.

The invention can be implemented in refrigerator, or any cooling device. The invention also has applicability in any device or machines, where change of state of the matter is concern, so that the change of state of the matter can be detected and controlled.

Thus, the present invention provides for a system 1 for detecting change of a state 2 of a matter contained in a container 3. The system 1 comprising a holder 4 for holding the container 3, a light emitting source 5 placed on or in proximity of a first wall 6 of the holder 4, and which generates a light 7, an optical sensor 8 placed on or in proximity of a second wall 9 of the holder 4, different from the first wall 6, such that the light emitting source 5 and the optical sensor 8 faces each other while in action. The optical sensor 8 receives the light 7 from the light emitting source 5 after passing through the container 3 and generates a lighting data 10 based on the light 7 received, wherein the container 3 is adapted to be placed in the holder 4, such that transparent parts of the container 3 faces the light emitting source 5 and the sensor 8. The system 1 also includes a processor 11 which receives the lighting data 10 and determines, if the state of the matter has changed.

### List of reference signs

- 1: system
- 2: state of matter
- 2a: first state of a matter
- 2b: second state of a matter
- 3: container
- 4: holder
- 5: light emitting source
- 6: first wall
- 7: light
- 8: optical sensor
- 9: second wall
- 10: lighting data
- 11: processor
- 12: alarm trigger
- 12a: first alarm trigger
- 12b: second alarm trigger
- 13: alarm
- 14: indication
- 15: control trigger
- 15a: first control trigger
- 15b: second control trigger
- 16: controller
- 17: cooling device
- 18: compartment
- 18a: cooling compartment
- 18b: state change compartment
- 19: cooling mechanism

## Claims

1. A system (1) for detecting a change of a state (2) of a matter contained in a container (3), the system comprising:
- a holder (4) for holding the container (3);
- a light emitting source (5) placed on or in proximity of a first wall (6) of the holder (4), the light emitting source (5) is adapted to generate a light (7);
- an optical sensor (8) placed on or in proximity of a second wall (9) of the holder (4), different from the first wall (6), such that the light emitting source (5) and the optical sensor (8) faces each other while in action, wherein the optical sensor (8) is adapted to receive the light (7) from the light emitting source (5) after passing through the container (3) and to generate a lighting data (10) based on the light (7) received, wherein the container (3) is adapted to be placed in the holder (4) such that transparent parts of the container (3) faces the light emitting source (5) and the sensor (8); and
- a processor (11) adapted to receive the lighting data (10) and adapted to determine, if the state of the matter has changed.

2. The system (1) according to the claim 1, wherein the processor (11) is adapted to generate an alarm trigger (12) based on the determination of the state (2) of the matter, the system (1) comprising:
- an alarm (13) adapted to receive the alarm trigger (12) from the processor (11), and adapted to generate an indication (14) indicating the change of the state (2) of the matter.

3. The system (1) according to any of the claims 1 or 2, wherein the processor (11) is adapted to generate a control trigger (15) based on the determination of the change of the state (2) of the matter, the system (1) comprising:
- a controller (16) adapted to receive the control trigger (15) and adapted to control change of the state (2) of the matter.

4. The system (1) according to any of the claims 1 to 3, wherein the matter is adapted to be transparent in a first state (2a), and to be translucent or opaque in a second state (2b).

5. The system (1) according to the claim 4, wherein the first state (2a) is a liquid state, and the second state (2b) is the solid state.

6. A cooling device (17) adapted to cool a matter, wherein the cooling device (17) comprising the system (1) according to any of the claims 1 to 5, the cooling device (17) further comprising a compartment (18a, 18b) having the holder (4) for keeping the container (3) containing the matter, and a cooling mechanism (19) to cool the matter contained in the container (3), wherein the matter is defined to have a physical property to change the state (2) when cooled beyond a particular temperature.

7. The cooling device (17) according to the claims 6 comprising at least two compartments (18a, 18b), a cooling compartment (18a) and a state change compartment (18b), wherein the cooling compartment (18a) is adapted to be cooled in a way by the cooling mechanism (19), so that the state (2) of the matter does not change, and the state change compartment (18b) is adapted to be cooled in a manner so that the change of the state (2) of the matter occurs within a predefined time interval.

8. The cooling device (17) according to the claim 7, wherein the processor (11) is adapted to generate a first alarm trigger (12a), if the state (2) of the matter has changed in the cooling compartment (18a), and the alarm (13) is adapted to receive the first alarm trigger (12a) from the processor (11), and is adapted to generate the indication (14) indicating the change of the state (2) of the matter.

9. The cooling device (17) according to any of the claims 7 or 8, wherein the processor is adapted to generate a second trigger alarm (12b), if the state (2) of the matter inside the state change compartment (18b) does not change in the predefined time interval, and the alarm (13) is adapted to receive the second alarm trigger (12b) from the processor (11), and adapted to generate the indication (14) indicating no change in the matter in the predefined interval.

10. The cooling device (17) according to any of the claims 7 to 9, wherein the processor (11) is adapted to generate a first control trigger (15a), if the state (2) of the matter has changed in the cooling compartment (18a), and the controller (16) is adapted to receive the first control trigger (15a), and adapted to regulate the cooling mechanism (19) to reverse the change of the state (2) of the matter.

11. The cooling device (17) according to any of the claims 7 to 10, wherein the processor (11) is adapted to generate a second control trigger (15b), if the state (2) of the matter inside the state change compartment (18b) does not change in the predefined time interval, and the controller (16) is adapted to receive the second control trigger (15b), and adapted to regulate the cooling mechanism (19) to enhance cooling inside the state change compartment (18b) to fasten the change of state (2) of the matter.
